# EUROPEAN PATENT APPLICATION

(11) **EP 4 807 755 A1**
(43) Date of publication of application: **16.09.2026**
(21) Application number: 25305324.3
(22) Date of filing: 12.03.2025
(51) Int. Cl.: G16H 15/00, G16H 30/40

(54) **AUTOMATED GENERATION OF A MEDICAL REPORT FROM ONE OR MORE ORGAN IMAGES**

(71) Applicant: Dassault Systèmes, 78140 Vélizy-Villacoublay (FR)
(72) Inventor: NDOKO, Arthur, 78140 Vélizy-Villacoublay (FR); SIMON, Aloïs, 78140 Vélizy-Villacoublay (FR)
(74) Representative: Bandpay & Greuter

(57) **Abstract**

The disclosure notably relates to a computer-implemented method for generating, from one or more medical images each of an organ of a patient, a medical report compliant with a predefined set of medical report rules. The method comprises, automatically, determining positions and diameters of one or more lesions in the one or more medical images. The method further comprising, automatically, determining, based on determined positions and diameters of the lesions, a list of target lesions in accordance with the predefined set of medical report rules. The method further comprises, automatically, generating the medical report based on the determined target lesions and their positions and diameters.

## Description

### TECHNICAL FIELD

The disclosure relates to the field of computer programs and systems, and more specifically to a method, system and program for generating, from one or more medical images each of an organ of a patient, a medical report compliant with a predefined set of medical report rules.

### BACKGROUND

In current practices, specialists in medical imaging diagnostics usually must produce a textual report describing the evaluation of quantitative response criteria (e.g., RECIST, WHO, Lugano) that they perform on the basis of imaging data. Generally, these reports include at least:
- A description of the findings of interest (e.g., tumorous lesions),
- Their measurements (e.g., diameters) and classification (e.g., target/non-target),
- The results of calculations evaluated from the measurements (e.g., the sum of longest target lesion diameters for RECIST).

In traditional practices, all of these elements are obtained and written manually. In other words, the expert medical imaging examinator detects the findings of interest in the medical images, measures them using digital tools by hand and classify them, computes the calculations mentally or using a calculator and finally, types all of these elements in a digital text document.

This represents a time-consuming workflow for specialists in medical imaging diagnostics. In addition, it induces variability both in the content and the format of the produced reports. This makes downstream automatic processing of these reports more complex and limited (e.g., aggregated analysis, patient feature extraction).

Within this context, there is still a need for improved solutions for generation of medical reports based on organ images of a patient.

### SUMMARY

It is therefore provided a computer-implemented method for generating, from one or more medical images each of an organ of a patient, a medical report compliant with a predefined set of medical report rules. The method comprises, automatically, determining positions and diameters of one or more lesions in the one or more medical images. The method further comprising, automatically, determining, based on determined positions and diameters of the lesions, a list of target lesions in accordance with the predefined set of medical report rules. The method further comprises, automatically, generating the medical report based on the determined target lesions and their positions and diameters.

The method may comprise one or more of the following:
- the predefined set of medical report rules corresponds to the RECIST standard or standards derived from the RECIST standards;
- the target lesions are determined according to the following rules:
   ∘ lesions with a large diameter are prioritized;
   ∘ target lesions must have a diameter larger than a predefined threshold;
   ∘ each organ in the images must be represented by at least one target lesion;
   ∘ maximum two lesions per organ; and
   ∘ maximum 5 target lesions;
- generating the medical report comprises generating an intermediary structured representation of the medical report;
- the intermediary structured representation is a DICOM SR document;
- generating the medical report further comprises converting the intermediary structured representation into a text format;
- converting the intermediary structured representation into the text format comprises filling a predefined report template based on the intermediary structured representation, wherein optionally the template comprises text sections and sections to add medical image previews with links to the original medical images;
- the method further comprises, prior to the generating of the medical report, by a user, modifying the list of target lesions;
- the method further comprises, prior to determining the list of target lesions, associating each lesion with a respective organ;
- associating each lesion with a respective organ comprises superimposing the positions of the lesions with a 3D map of the organs; and/or
- the images are CT-scans or MRIs.

It is further provided a data structure comprising a medical report obtained according to the method. The data structure may be any file storing the medical report outputted by the method, such as, for example, a docx file or pdf file into which the report outputted by the method has been exported.

It is further provided a computer program comprising instructions for performing the method.

It is further provided a computer readable storage medium having recorded thereon the computer program and/or the data structure.

It is further provided a system comprising a processor coupled to a memory (and optionally to a graphical user interface), the memory having recorded thereon the computer program and/or the data structure.

It is further provided a device comprising a data storage medium having recorded thereon the computer program and/or the data structure.

The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (e.g. the device is a subsystem of the overall system). The system may further comprise a graphical user interface coupled to the processor.

### BRIEF DESCRIPTION OF THE DRAWINGS

Non-limiting examples will now be described in reference to the accompanying drawings, where:
- FIG.s 1 to 6 illustrate the method; and

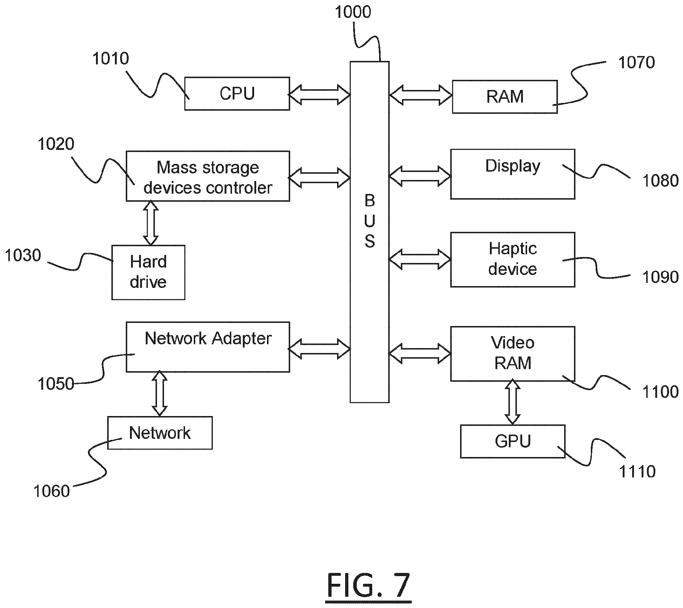

- FIG. 7 shows an example of the system.

### DETAILED DESCRIPTION

It is therefore proposed a computer-implemented method for generating, from one or more medical images each of an organ of a patient, a medical report compliant with a predefined set of medical report rules. The method comprises, automatically, determining positions and diameters of one or more lesions in the one or more medical images. The method further comprising, automatically, determining, based on determined positions and diameters of the lesions, a list of target lesions in accordance with the predefined set of medical report rules. The method further comprises, automatically, generating the medical report based on the determined target lesions and their positions and diameters.

This constitutes an improved solution for generation of a medical report based on organ images of a patient.

First, the claimed method fully automates the process of inferring a medical report following given medical report rules from one or more medical images, i.e. fully automates mandatory steps of determining the positions and diameters of the lesions, determining the target lesions, and generating the report. Indeed, the method starts (takes as input) one or more images each of an organ of a patient (*e.g.* CT-scan image or MRI images and automatically determines positions and diameters of lesions in these images, automatically finds target lesions following the given medical report rules, and automatically generates a medical report based on the target lesions (and their computed positions and diameters). A medical report following a predefined set of medical report rules (*e.g.* RECIST, of the like), is thus automatically extracted from organ medical images (*i.e.* images which stem from a medical imaging process, such as a CT-scan or a MRI). In other words, the method provides automatically a written description (the report) of the medical state of the patient as captured by measured data (the images), and this description follows medical/scientific standards (the predefined rules). As further discussed hereinafter, this automation however does not exclude further steps performed by the user, for example for triggering the automated steps and/or for validation/correction.

The method thus provides a fully automated workflow from the medical image data to the textual report. The method may comprise steps related to approval or correction provided by the relevant specialist/practitioner, but the proposal of the report is automatic. Therefore, the production of the report is much faster on average. For example, it makes it possible to generate the text report in advance and let the specialists in medical imaging diagnostics only validate them or correct them. This may significantly accelerate the production of reports for a cohort of patients even in a case where only a minority of the automatically generated reports would be validated without modification.

Furthermore, the entire workflow is deterministic which allows to greatly reduce the variability of the generated reports both in terms of format (e.g., same text structure, vocabulary choices, number formats, etc.) and content (e.g., choice of target lesions for RECIST) even when the specialist in medical imaging diagnostics may modify them considering that the first suggestions would influence them. It also reduces the risk of human mistakes, typically, for the computation of the quantities of interest.

The method is for generating, from one or more medical images each of an organ of a patient, a medical report. This means that the method takes as input the image(s) and outputs the report (*e.g.* as any suitable text file, such as a .docx or a .pdf file). The method thus outputs data encoding a medical report, which is a medical description of a condition/state of the patient as captured by the input image(s). The medical report outputted by the method is compliant with a predefined set of medical report rules. This means that the medical report is drafted (automatically by the method) according to these rules and follows them. The medical report may for example be drafted by filling a predefined template, which follows these rules, by specific patient data that stems from the medical images and their processing by the method. The medical report also follows the rules in that it concerns the target lesions which have been automatically selected based on these rules. Any patient herein may be a patient having cancer, treated for cancer, and/or having suspicion cancer. Each image of that patient is in this case an image of an organ relevant for the cancer, *i.e.* on which lesions are found or likely to be found. Any lesion herein may thus be a tumorous lesion. The medical report may thus be an oncology report, and the set of rules may relate to that field (*e.g.* they may correspond to the RECIST standard or derivatives thereof in the field of oncology).

The one or more medical images are all respective to a same given patient. Each image is an image of an organ of the patient. All images may be relative to the same organ of the patient (for example, they may correspond to different views of that organ). Alternatively, the images may consist of images of several organs of the patient (which does not exclude several of the images from being relative to a same organ). The patient is a human patient. "Medical image" means an image of a portion of the body of the patient (*i.e.* the organ or the organ and a vicinity thereof), the image having been acquired by a medical imaging process. All the images of said one or more images are of the same type, *i.e.* they have been acquired by the same medical imaging process. The medical imaging process may be a CT-scan, in which case each medical image is a CT-scan image. The medical imaging process may alternatively be a MRI, in which case each image may be a MRI image. The medical imaging process may alternatively be a PET scan, in which case each image may be a PET scan image The method may comprise an initial step of obtaining the one or more medical images. This may comprise performing the medical imaging process to acquire the image(s). Alternatively, this may comprise obtaining (*e.g.* downloading) the already acquired images from a (*e.g.* remote) memory or server or database where they have been stored further to their acquisition by the imaging process.

The method comprises, automatically, determining positions and diameters of one or more lesions in the one or more medical images.

Determining the positions and diameters of the one or more lesions may comprise applying, to each image, a neural network configured for (*i.e.* trained for) detection of the boundaries of the lesions in the image (which may be referred to as "segmentation" of the image). This neural network takes as input the image and outputs localized representations each of a lesion in the image, for example output bounding boxes and/or segmentation mask each around a lesion. This neural network is trained on a dataset adapted for this purpose, *i.e.* a dataset of training examples, each training example comprising: 1) a medical image comprising one or more lesions and 2) ground truth localized representations each one of these lesions. The dataset of training examples may optionally comprise negative training examples, *i.e.* medical images with no lesions (having for example a ground truth negative label or the like). The dataset is adapted to the task at hand: for example, if the task is to establish an oncology medical report based on CT-scans, the dataset is formed by CT-scans showing tumorous lesions. The dataset comprises a sufficient variability and quantity (*e.g.* more than a thousand) of examples for the task at hand (*e.g.* CT-scans with tumorous lesions for an oncology report), as known *per se* from machine-learning. Training the neural network comprises, as known *per se,* feeding the training examples to the neural network and minimizing a loss that penalizes, for each training example, a disparity between the detection made by the neural network in the image of the training example and its corresponding ground truth data. The loss may be, for example, the DICE loss. The neural network has an architecture adapted for this segmentation/detection task, for example the U-Net architecture (discussed in reference O. Ronneberger, P. Fischer, and T. Brox, "U-Net: Convolutional Networks for Biomedical Image Segmentation," in Medical Image Computing and Computer-Assisted Intervention - MICCAI 2015, N. Navab, J. Hornegger, W. M. Wells, and A. F. Frangi, Eds., in Lecture Notes in Computer Science. Cham: Springer International Publishing, 2015, pp. 234-241. doi: 10.1007/978-3-319-24574-4_28, which is incorporated herein by reference), the Mask R-CNN architecture (discussed in reference K. He, G. Gkioxari, P. Dollar, and R. Girshick, "Mask R-CNN," presented at the Proceedings of the IEEE International Conference on Computer Vision, 2017, pp. 2961-2969. Accessed: Jan. 11, 2022. [Online]. Available: https://openaccess.thecvf.com/content iccv 2017/html/He Mask R-CNN ICCV 2017 paper.html, which is incorporated herein by reference), or the UNETR architecture (discussed in reference A. Hatamizadeh et al., "UNETR: Transformers for 3D Medical Image Segmentation," in 2022 IEEE/CVF Winter Conference on Applications of Computer Vision (WACV), Waikoloa, HI, USA: IEEE, Jan. 2022, pp. 1748-1758. doi: 10.1109/WACV51458.2022.00181, which is incorporated herein by reference). The method may comprise an initial stage (offline stage) of training the neural network or may simply use an already trained neural network for determining the lesions in the images. Determining the positions and diameters of the one or more lesions may further comprise, after application of the neural network, performing a post-processing of the output of the neural network, for example smoothing the segmentation masks, or aligning the bounding boxes long the Z-axis. The output of the neural network in any case describes the positions of the lesions in the images.

The method may optionally comprise a step of providing to the user (*e.g.* a practitioner) the results outputted by the neural network, for example by displaying them (*e.g.* displaying all the images with visual representations of the bounding boxes or segmentation masks) to the user. The user may be offered the possibility (for example through graphical user interaction) of validating or modifying these results. Alternatively, these steps related to validation of the output of the neural network may not be performed by the method (which does not exclude further validation steps downstream).

Determining the positions and diameters of the lesions may then comprise, for each lesion detected by the bounding box, automatically computing its diameter. This may be done by any suitable diameter computation method, for example by computing the diameter as the distance between the most distant pair of points in the outputted position description of the lesion by the neural network (*e.g.* most distant pair of points in the bounding box outputted by the neural network for this lesion)

The method may then comprise, in addition to the determining of the positions and diameters of the lesions and prior to determining the list of target lesions, automatically labeling each lesion with an organ to which the lesion correspond (*i.e.* labeling the lesions with the name or other suitable ID of the organ on which the lesion is located). In other words, the method may further comprise, prior to determining the list of target lesions, associating each lesion with a respective organ. Associating each lesion with a respective organ may comprise superimposing the positions of the lesions with a 3D map of the organs. This may be done by applying a predefined segmentation mask to the medical images (or segmentation method or algorithm) configured to detect the class of each organ (*e.g.* name or ID of the organ: heart, liver, aorta, etc.). Such masks are known from the prior art and are already available, and this step of the method may thus use such available segmentation mask. For example, the method may apply the Total Segmentator model (discussed in reference J. Wasserthal et al., "TotalSegmentator: Robust Segmentation of 104 Anatomic Structures in CT Images," Radiol. Artif. Intell., vol. 5, no. 5, p. e230024, Sep. 2023, doi: 10.1148/ryai.230024, which is incorporated herein by reference) or any similar deep neural network. Further to the obtention of the organ classes with such mask, the classifying may perform the labeling by identifying the intersections between the determined positions of the lesions and this obtained segmentation mask: when a lesion intersects a mask of an organ, the lesion is assigned with the label/class of that organ.

Alternatively, the labeling of the lesions with the organ labels may be performed simultaneously to the determination of the positions of the lesions. For example, the method may apply the MULAN deep neural network (discussed in K. Yan et al., "MULAN: Multitask Universal Lesion Analysis Network for Joint Lesion Detection, Tagging, and Segmentation," in Medical Image Computing and Computer Assisted Intervention - MICCAI 2019, D. Shen, T. Liu, T. M. Peters, L. H. Staib, C. Essert, S. Zhou, P.-T. Yap, and A. Khan, Eds., in Lecture Notes in Computer Science. Cham: Springer International Publishing, 2019, pp. 194-202. doi: 10.1007/978-3-030-32226-7_22, which is incorporated by reference), which takes as input each image, an output localized representations each of a lesion in the image together with the label of the organ to which the lesion belongs.

As output of the step of determining the positions and diameters of the lesions, the method may thus yield a list of lesions each associated with its computed diameter and a label indicating the organ to which the lesion belongs.

The method further comprises automatically determining, based on the determined positions and diameters of the lesions, a list of target lesions in accordance with the predefined set of medical report rules. This may be done by any algorithm which browses the determined lesions and classifies them by following the rules of the predefined set.

For example, the predefined set of medical report rules may correspond to the RECIST standard, or standards derived from the RECIST standards, such as mRECIST or iRECIST. The rules thus include in this case: 1) rules for filling the medical report, and 2) rules for selection of the target lesions. The determining of the target lesions follows these rules 2) for selection of the target lesions, if the rules correspond to the RECIST standard (or its derivatives). The set of rules 2) according to which the target lesions are determined (selected) may for example be:
- lesions with a large diameter are prioritized (*e.g.* the lesions may be classified according to an increasing order of their diameter and the lesions classified first tend to be priority for the selection (while accounting for the remaining rules));
- target lesions must have a diameter larger than a predefined threshold (the threshold may for example equal 10mm, as for the RECIST standard, the non-lymphatic lesions may be required to have a diameter larger than 10mm to be measurable and thus potential targets);
- each organ in the images must be represented by at least one target lesion (*i.e.* the images altogether comprise representation of one or more organs, and each organ must have a lesion selected in the target lesions);
- maximum two lesions per organ; and
- maximum 5 target lesions.

Selecting this set of rules may for example be done by the algorithm described by the following pseudo-code:

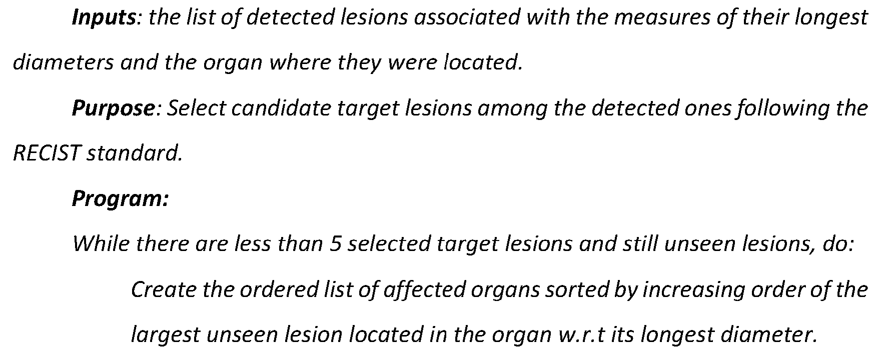

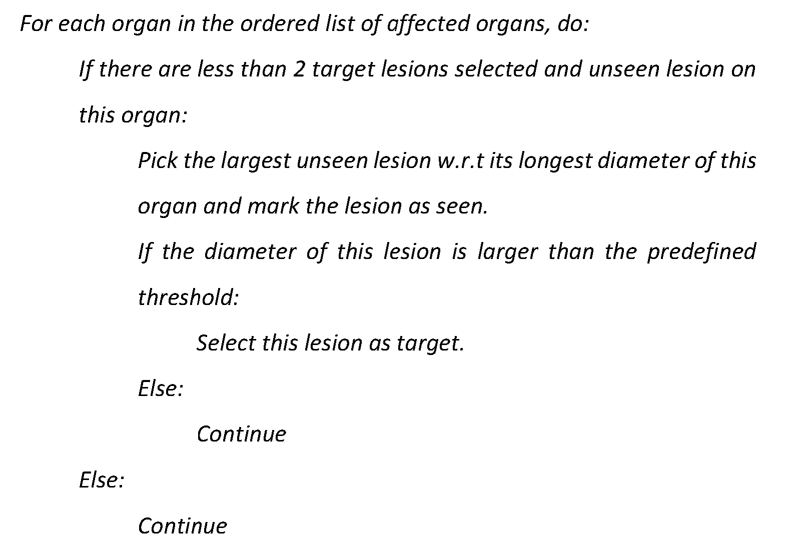

N.B: The rule according to which each organ must be represented may be subordinate to the one that limits the number of lesions to 5 (if 6 organs are affected, only 5 lesions remain). From then on, at each iteration of the first loop while, the algorithm goes through the list of affected organs and mark as target the largest lesion "not already seen" for each of these organs. In other words, on each of these iterations, the algorithm goes through all the organs and add a maximum of 1 lesion per organ, thus ensuring that the algorithm cannot add a second target lesion for a given organ without first adding the potential target lesions for the other organs.

Additional rules for selecting the target lesions may optionally, thus not necessarily, be used, such as discarding touching lesions and/or prioritizing lesions with well-defined borders (by summing the amplitude of the image gradient on the segmentation boundaries).

Again, the output of the classifying of the findings of interest may be displayed to the user (*e.g.* a specialist in medical imaging diagnostics) for validation or correction in an ad-hoc graphical interface that allows to quickly validate or correct a classification (for example, via keyboard interactions). Alternatively, the user may only perform the validation and corrections once the textual report is generated.

The method may further comprise, prior to the generating of the medical report, by a user (typically a practitioner), modifying the list of target lesions. For example, the method may comprise providing (*e.g.* displaying) to the user the list of target lesions together with the positions, diameters and/or labels of the lesions (for example with this data being added to the corresponding medical images), for validation or correction (*e.g.* in an ad-hoc graphical interface) that allows to quickly validate or correct a classification (for example, via keyboard interactions). Alternatively, the specialist in medical imaging diagnostics may only perform the validation and corrections once the report is generated.

The method then comprises automatically generating the medical report based on the determined target lesions and their positions and diameters. Generating the medical report means writing the medical report, by following the predetermined set of rules, the medical report being written using the determines target lesions and their positions and diameters.

Generating the medical report may comprise generating (automatically) an intermediary structured representation of the medical report. Thus, the relevant data about the lesions (positions, diameters) in the images may be put in a structured representation, prior to generating a text medical report. The intermediary structured representation may be a DICOM SR document (*e.g.* stored as one or more SR files). In this case, generating the intermediary structure representation (*i.e.* the DICOM SR document) may comprise automatically providing a template structured document, which has a structure compliant with the predefined set of rules (*e.g.* the RECIST standard or its derivatives).

The template DICOM SR document for the RECIST standard may for example have the following structure:
1. **Document title:** IMAGING MEASUREMENT REPORT
2. **Date**
3. **Language:** ENGLISH
   1. **Country of Language**
4. **Observer context :**
   1. **ObserverType:** PERSON
   2. **ObserverName:** Defaults to "UNKNOW"
5. (Facultative)**ProcedureReported**
6. **Imaging measurements**
   1. **Measurement Group** *(one measurement group per lesion)*
      i. **Tracking identifier**
      ii. **Tracking unique identifier**
      iii. **Time Point Identification** *(Baseline, nadir, etc.)*
      *iv.* **Finding Category:** *(Indicate target*/*non-target etc.)*
      v. **Measurement method:** RECIST
      *vi.* **Finding Site:** *(Organ name)*
      vii. **Laterality** *(if relevant)*
      viii. **Algorithm Identification**
         **1. Algorithm Name**
         **2. Algorithm Version**
         *3. (Facultative)* **Algorithm Parameter**
         *4. (Facultative)* **Algorithm Family**
      ix. **Measurement**
         1. **Num:** *(The measured diameter. Concept Name: "Long axis" (SCT: 10339001) or "Short axis" (SCT: 103340004))*
            I. **Units:** mm
            II. **Number**
         **2. Inferred from**
            I. **Selected from** *(Image, includes an icon image)*

Generating the intermediary structured document (*e.g.* the DICOM SR document) may then comprise automatically filing the template with the data of the images and the lesions. For example, filing the above template for a DICOM SR document may comprise:
- Determining and writing in the "language" section the language and country of language from the images, by using any suitable method (*e.g.* text extraction model or algorithm). Alternatively, the language may be fixed (*e.g.* mandatory English). "Country of language" may thus in this case be used to specify whether the English is US English or UK English. Yet alternatively, the "country of language item may be optional or not even used ;
- Setting (in the "date" section) the date to the current date (retrieving for example these data from the computer system);
- Determining the name or ID of the observer and writing it in the "Observer context" section, for example by retrieving the name of the user of the computer (*e.g.* from computer session data). If this determination is not possible or if the observer is unknown (*e.g.* not identified on the computer), the observer name or ID is set to "unknown", which is the default option;
- Determining, from the images or other data (*e.g.* patient file), whether a procedure is reported, and writing the procedure (if determined) in "ProcedureReported". If there is no procedure nothing is written in this section;
- Determining (and writing in the appropriate line of each "measurement group" section (one such section per lesion)), for each lesion,
   ∘ an identifier of the lesion and a unique identifier of the lesion. The identifier may be a free identifier (typically "lesion 1", "lesion 2", etc.) Determining the identifier may thus comprise assigning to each lesion the identifier "lesion n" where for example n refers to the order of identification of the lesion (*i.e.* n= 1 means that the lesion is the first identified lesion) or n may for example be an ID of a bounding box determined around the lesion as discussed above. The unique identifier may be an identifier selected so that it identifies uniquely the lesion and so that the risk of duplicate unique identifier with another lesion is avoided or at least reduced. For example, that unique identifier may be a random number with numerous digits (*e.g.* of the type 14680543.54087421.23035347) and determining that unique identifier may thus comprise automatically generating such random number.
   o the time point identification of the lesion. The time point corresponds to the point of time to which the image corresponds and may thus be extracted automatically from the image. The time point may for example indicate "baseline", which means "before a treatment of the patient", or baseline + a certain time, or "nadir", which means "the exam for which the smallest SLD has been measured during the course of the patient's follow-up. The time point may be automatically extracted from the corresponding medical image, which comprises data describing the time of acquisition of the image. ∘ whether the lesion has been classified as target lesion or not;
   ∘ Measurement method is RECIST by default;
   ∘ the finding site as the organ name, using for example the determined organ label associated with the lesion:
   ∘ the laterality (for organs organized in pair, such as the kidneys or the lungs), which can be left or right, and may be automatically determined from the image or data describing the image;
   ∘ data about the algorithm used for measuring the diameters, *e.g.,* by retrieving these data from the algorithm description data, including name, version, optionally parameters (if any) and family (if any);
   ∘ for the "measurement" section, the concept name of the measured diameter (long axis or short axis), the number (*i.e.* the measured value, in mm which is set fixed as the default unit), and the image from which the measurements stems (including an image ID, and optionally a link to the image and/or an icon image representing the image)

For each lesion, the "measurement" section comprises the measurement of the long axis of the lesion, which is the diameter of the lesion computed by the method, *i.e.* the largest diameter as previously discussed. The section may also comprise the measurement of the short axis as the lesion, which the method may compute for example after computation of long axis, as the longest line (within the lesion (*i.e.* touching the boundary of the lesion at both ending points of the line) perpendicular to the long axis.

Generating the medical report may further comprise automatically converting the intermediary structured representation into a text format. From the point of view of the user, the generation of the intermediary structured representation and the conversion into a text format may be transparent, *e.g.* it may be done as a background process, and the user only sees the final result, *i.e.* the text format. The conversion into the text format may comprise automatically reading the intermediary structured representation, extracting data therein, and automatically writing these data as a text format medical report. Converting the intermediary structured representation into the text format may comprise automatically filling a predefined report template based on the intermediary structured representation (*i.e.* based on extracting the data therein). The template comprises text sections and sections to add medical image previews with links to the original medical images. The predefined textual report template may be defined using predefined placeholders and conditional blocks (established in accordance with selected quantitative response criterion and needs of the specialists in medical imaging diagnostics). The automatic filling of the predefined textual template is eased by the structuration of all the necessary information, *i.e.* the use of an intermediate structured representation. The placeholders may be replaced automatically with the corresponding information in the data structure and text blocks may be added depending on conditions and the number of findings. The textual report may contain hyperlinks enabling to display the referred medical images in the medical image viewer. Typically, the structure of the report template may be encapsulated within a dedicated computer-implemented function that takes as input the automatically extracted data. Once the filling is complete the automatic draft textual report is human-readable, and it can be submitted to the specialist in medical imaging diagnostics for validation and correction. The specialist in medical imaging diagnostics may correct the final textual report by amending directly its content using a digital text editor. The digital text editor may be integrated as a tool in the medical imaging viewer.

For example, if the set of rules correspond to the RECIST standard, the predefined textual report template may contain blocks like (where [] denotes placehoders):
- *"> Target Lesion [lesion_id_number]: located in the [lesion_organ_location] with a long axis diameter of [long_axis_diameter] mm, see [image_link]."*
- *"Sum of longest diameters: [sld] mm."*

Let us assume that the following data has been extracted automatically from the medical imaging acquisition and structured as a dictionary:
- *{id: 1, classification:"target", organ: "right kidney", long_axis_diameter: 42.75, image_link: "pacs.slice_16.url"},*
- *{id: 2, classification:"target", organ: "liver", long_axis_diameter: 18.10, image_link: "pacs.slice_27.url"}*
- *{sld: "60.85"}*

Following the template block above, the output text report will then contain the following lines:
*"> Target Lesion 1: located in the right kidney with a long axis diameter of 42.75 mm see link.*
*> Target Lesion 2: located in the liver with a long axis diameter of 18.10 mm see link.*
*Sum of longest diameters: 60.85 mm."*

If the intermediary structured representation is the previously discussed DICOM SR document, the conversion of that DICOM SR document into a text format may be performed according to the algorithm described by the following pseudo- code:

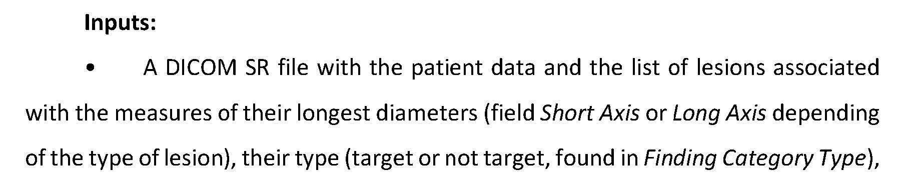

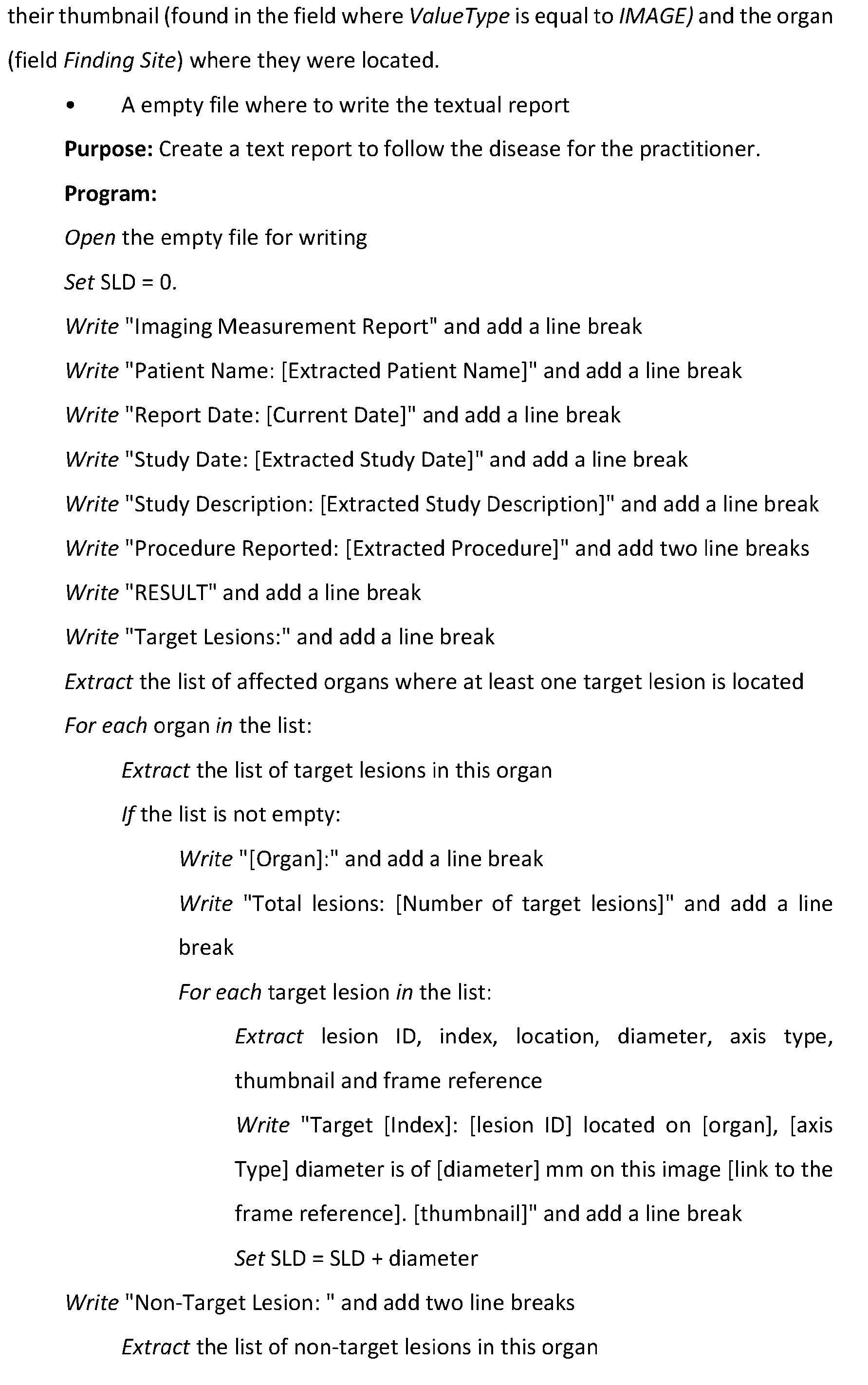

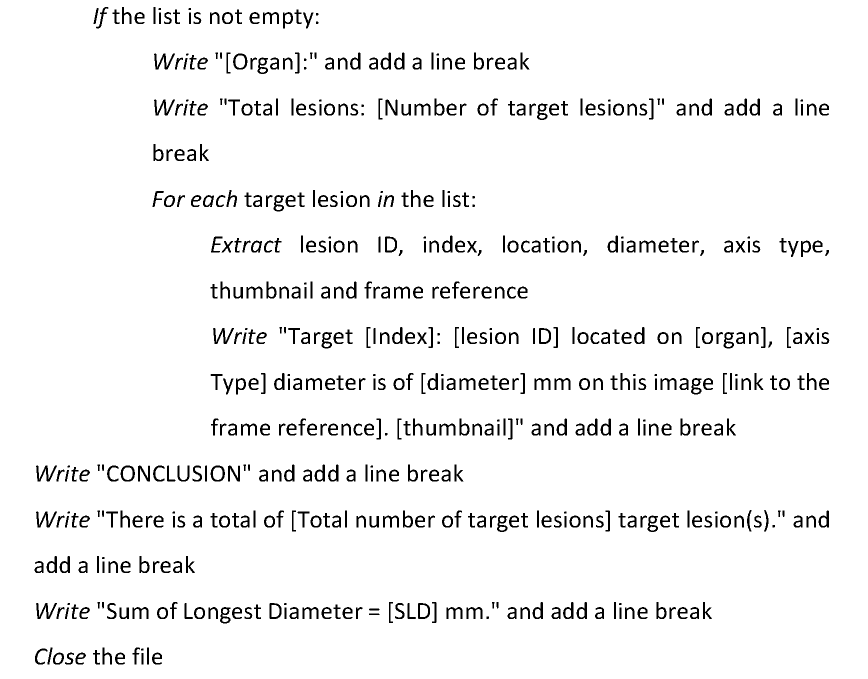

N.B.: In the above pseudo-code, "frame" refers to a medical image.

The text format is editable. This allows a user (*e.g.* a practitioner) to edit, correct, and/or complete the report, at least in part. The method may comprise such a step of editing the text report, by a user, for example by providing correction or completion.

Examples of scenario illustrating the method are now discussed.

A first typical example of scenario of use of the method is the following.

A radiologist is contracted as an off-site reader in the context of a clinical trial for cancer treatment. They must deliver 100+ RECIST reports of their readings of the CT-scans acquired during the clinical trial. This would take them at least 2 days of work with current practices. Instead, using the invention, they can launch the automatic generation of the reports during the night.

The report generation process utilizes a deep neural network (the previously discussed MULAN model for example) to detect tumorous lesions and classify in which organ they are located. The target lesions are automatically selected using adapted rules and the diameters and their sums are computed. All this information is converted into a DICOM Structured Report that serves as basis for the generation of the text report.

The radiologist has now only to review the generated reports and validate them. If no correction is necessary, this validation step costs significantly less time than a complete RECIST reading. Therefore, overall, radiologists can deliver the required RECIST reports faster.

Additionally, at least two readings are usually performed by different radiologists for the same medical acquisitions during a clinical trial. If a major discrepancy is detected for a medical acquisition, then a third radiologist may be mandated to perform the adjudication. As the workflow introduced by the method is deterministic, such discrepancies can be expected to be rarer.

FIG.s 1 to 5 are screenshots illustrating the use of method, implemented with a dedicated graphical user interface (GUI) allowing user interactions. FIG.s 1 to 5 show screenshots taken from this GUI. The images in this example are CT-scans, and the example still relates to the RECIST standard for the set of rules.

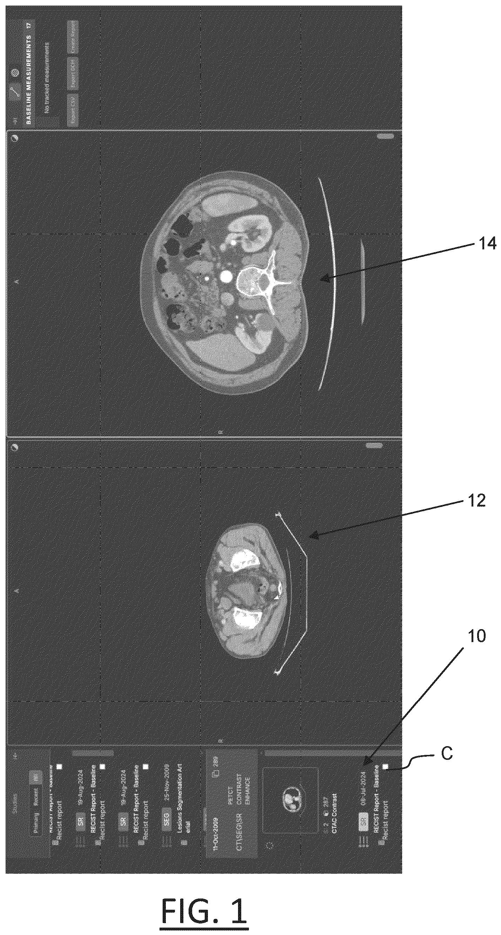

FIG. 1 shows a list of dates (19 august 2024 (twice), 25 November 2009, 8 July 2024) each corresponding to a respective subset of the set of medical images for a respective patient, said subset having been acquired at the indicated date. A small preview of each subset may be displayed (for example by hovering over the corresponding date, or near it). FIG. 1 shows that a small preview 10 of the images dated 08 July 2024 is shown, and images (here two images, 12 and 14) of this subset are then displayed in bigger views. As can be seen on FIG. 1, a checkbox is graphically selectable (by click or touch on the checkbox, as known in the art) for each date. Reference C corresponds to the checkbox for date 08 July 2024.

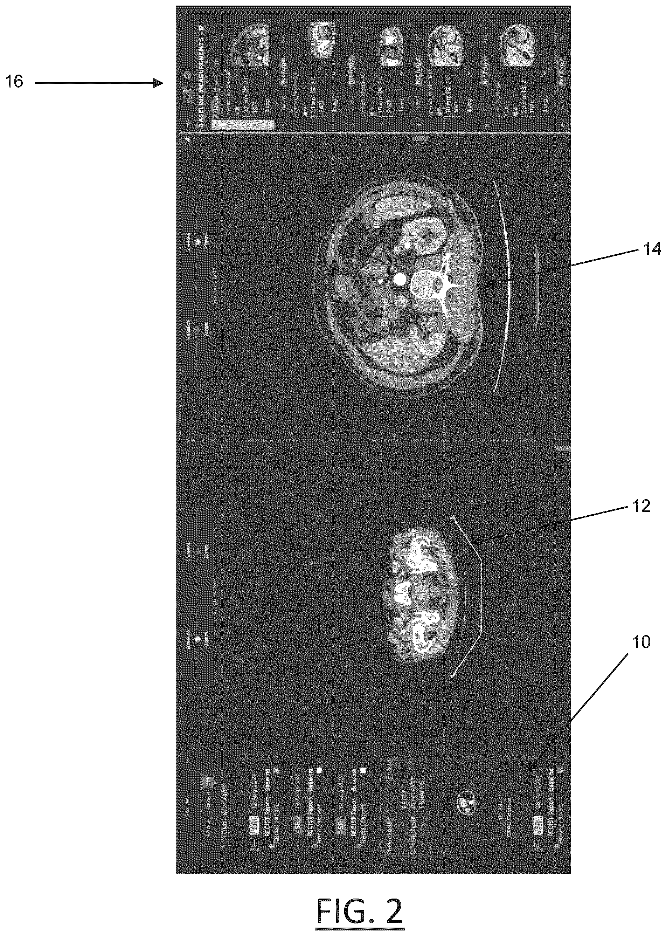

FIG. 2 shows that the user has selected the checkboxes for dates 08 July 2024 and 13 august 2024. This triggers automatic execution of the determining of the positions and diameters of the lesions in all the images corresponding to these dates, and the automatic execution of the determining of the target lesions. As can be seen in the figure the images are as a result displayed together with graphical representations of the lesions therein and their measured diameters. The time of the image can be set by a graphical widget, as can be seen on the figure, which allows to select the baseline time or a subsequence time (here 5 weeks for image 14). Modifying the selected time may lead to automatic recalculation of the lesions, their diameters, and the list of targets. The list 16 of the lesions is also displayed, each with a preview of the lesion, its diameter, and a classification target or non-target.

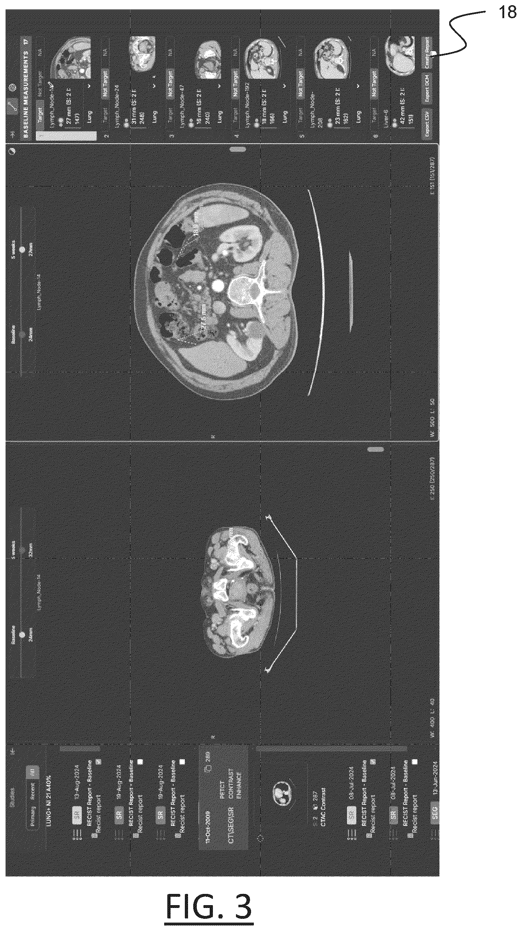

As it can be seen on FIG. 3, an icon "create report" 18 is displayed and may be graphically selected by a user (by a touch or click on the icon, as known *per se*)*.* This selection triggers automatically the step of automatically generating the report.

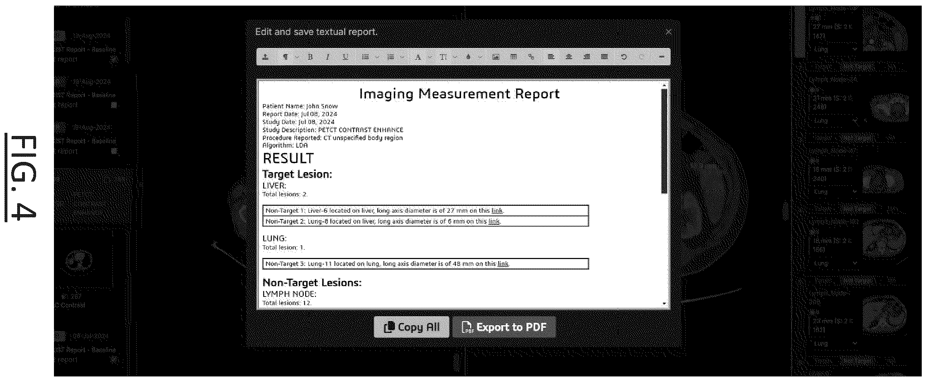

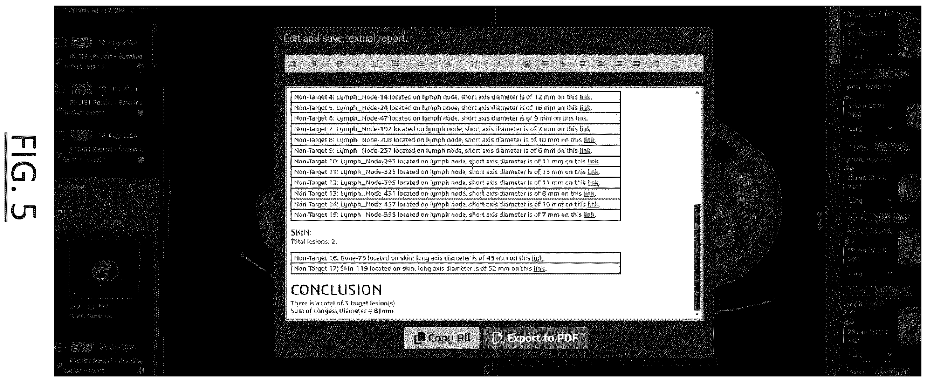

FIG. 4 shows a display of the created textual report. As can be seen, the report is editable, contains the relevant data extracted from the images, and links to the relevant images. Two icons "Copy all" and "Export to PDF" are graphically selectable by the user (by touch or click) and respectively allow to copy all the report (for further edition or use in another software) or to export it as a PDF file. Although not shown in the figure, exportation in other formats than PDF, using similar icons, may be proposed. The report is partially shown on FIG. 4, but the user can scroll down (using the mouse scroll wheel or the like, or the side scrolling bar). FIG. 5 shows a result of scrolling down, with the end of the textual report being shown.

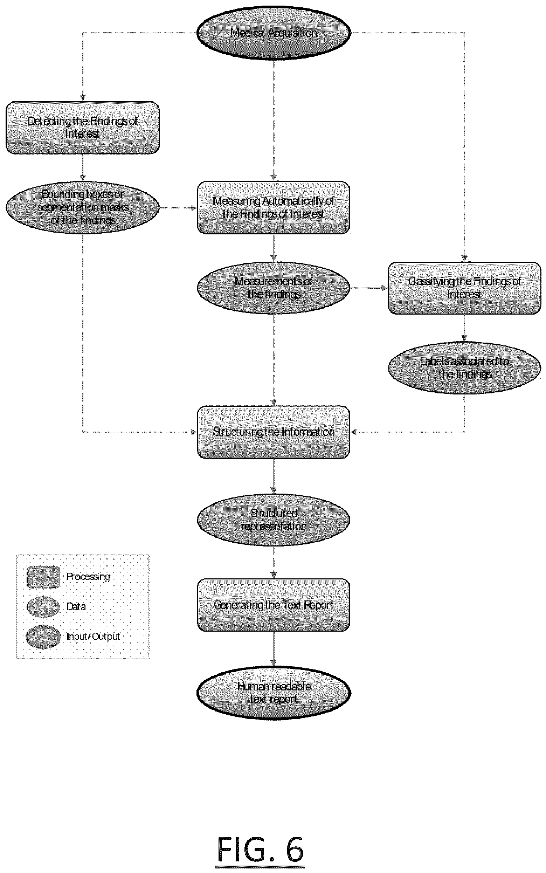

FIG. 6 shows a flowchart of implementations of the method.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

As previously discussed, each of the step of determining the positions and diameters of the lesions, the step of determining the target lesions, and the step of generating the report are performed automatically. The steps may be all performed automatically without user actions between the steps, for example upon the user launching the method (*e.g.* by clicking or touching an icon for launching the method). Alternatively, any one or each of these steps may be performed automatically but upon triggering of the automatic execution of the step by the user, for example by clicking or touching a specific icon. This was for example the case in the example of FIG.s 1 to 5 discussed above. Furthermore, as previously discussed, any one or each of these steps of the method (*i.e.* determining the position and diameter of the lesions, determining the target lesions, and generating the report) may be followed by a step of displaying the (intermediary) results to the user for validation or correction, as previously discussed. The word "automatically" in the method thus does not limit the method to the case where the three steps (*i.e.* determining the position and diameter of the lesions, determining the target lesions, and generating the report) are performed automatically without any user action between them (although this implementation is possible), but is to be understood as meaning that each of these step does not require user action, even if each step may be triggered by a user and/or followed by a validation/correction step as previously explained.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

FIG. 7 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer-implemented method for generating, from one or more medical images each of an organ of a patient, a medical report compliant with a predefined set of medical report rules, the method comprising, automatically:
- determining positions and diameters of one or more lesions in the one or more medical images;
- determining, based on the determined positions and diameters of the lesions, a list of target lesions in accordance with the predefined set of medical report rules; and
- generating the medical report based on the determined target lesions and their positions and diameters.

2. The method of claim 1, wherein the predefined set of medical report rules corresponds to the RECIST standard or standards derived from the RECIST standards.

3. The method of claim 2, wherein the target lesions are determined according to the following rules:
- lesions with a large diameter are prioritized;
- target lesions must have a diameter larger than a predefined threshold;
- each organ in the images must be represented by at least one target lesion;
- maximum two lesions per organ; and
- maximum 5 target lesions.

4. The method of any one of claims 1 to 3, wherein generating the medical report comprises generating an intermediary structured representation of the medical report.

5. The method of claim 4, wherein the intermediary structured representation is a DICOM SR document.

6. The method of claim 4 or 5, wherein generating the medical report further comprises converting the intermediary structured representation into a text format.

7. The method of claim 6, wherein converting the intermediary structured representation into the text format comprises filling a predefined report template based on the intermediary structured representation, wherein optionally the template comprises text sections and sections to add medical image previews with links to the original medical images.

8. The method of any one of claims 1 to 7, wherein the method further comprises, prior to the generating of the medical report, by a user, modifying the list of target lesions.

9. The method of any one of claims 1 to 8, wherein the method further comprises, prior to determining the list of target lesions, associating each lesion with a respective organ.

10. The method of claim 9, wherein associating each lesion with a respective organ comprises superimposing the positions of the lesions with a 3D map of the organs.

11. The method of any one of claims 1 to 10, wherein the images are CT-scans or MRIs.

12. A data structure comprising a medical report obtained according to the method of any one of claims 1 to 11.

13. A computer program comprising instructions which, when executed by a computer system, cause the computer system to perform the method of any one of claims 1 to 11.

14. A computer-readable storage medium having recorded thereon the computer program of claim 13 and/or the data structure of claim 12.

15. A computer system comprising a processor coupled to a memory, the memory having recorded thereon the computer program of claim 13 and/or the data structure of claim 12.
